# EUROPEAN PATENT APPLICATION

(11) **EP 4 809 912 A1**
(43) Date of publication of application: **23.09.2026**
(21) Application number: 25165484.4
(22) Date of filing: 21.03.2025
(51) Int. Cl.: A61B 5/024, A61B 5/026, A61B 5/08, A61B 5/0295, A61B 5/00, G16H 50/20

(54) **COMPUTER-IMPLEMENTED METHOD, COMPUTER PROGRAM PRODUCT, COMPUTER-READABLE STORAGE MEDIUM AND SLEEP STAGING DEVICE**

(71) Applicant: Koninklijke Philips N.V., 5656 AE Eindhoven (NL)
(72) Inventor: VAN DER AAR, Jaap Floris, 5656 AG Eindhoven (NL); FERREIRA DOS SANTOS DA FONSECA, Pedro Miguel, 5656 AG Eindhoven (NL); VAN DEN ENDE, Daan Anton, 5656 AG Eindhoven (NL); PERI, Elisabetta, 5656 AG Eindhoven (NL); OVEREEM, Sebastiaan, 5656 AG Eindhoven (NL); VAN GILST, Merel Marietje, 5656 AG Eindhoven (NL)
(74) Representative: Philips Intellectual Property & Standards

(57) **Abstract**

There is provided a computer-implemented method for sleep staging of a subject, comprising: receiving a first measurement data representative of a first physiological property relating to the autonomic nervous system of the subject; providing a first sleep stage classification by using a first sleep stage classifier to classify sleep stages of the subject based on the first measurement data; providing a second sleep stage classification by using a second sleep stage classifier, wherein the second sleep stage classifier is different than the first sleep stage classifier; determining a difference between a first performance of the first sleep stage classifier and a second performance of the second sleep stage classifier; determining, based on the difference, an autonomic modulation value representative of an autonomic modulation of the subject, wherein the autonomic modulation represents a relationship between the autonomic nervous system and a central nervous system of the subject.

## Description

### FIELD OF THE INVENTION

The invention relates to a computer-implemented method for sleep staging of a subject. Further, the invention relates to a computer program product and a computer-readable storage medium comprising instructions which, when executed by a processor, cause the computer-implemented method to be carried out. Further, the invention relates to a sleep staging device adapted to perform sleep staging for a subject.

### BACKGROUND OF THE INVENTION

Sleep-disordered breathing (SDB) conditions, such as OSA and CSA, are becoming increasingly common, and are particularly prevalent in older people, people with a high body mass index, smokers, heavy drinkers and people with conditions such as coronary artery disease, hypertension and diabetes mellitus.

SDB conditions are often treated using positive airway pressure (PAP) therapy, in which pressurized air is provided to a subject to keep the subject's airways open. When first prescribing PAP therapy, a PAP titration study is carried out for the subject in order to determine a level of airway pressure to be provided to the subject during PAP therapy, as well as a suitable PAP therapy modality (e.g. continuous positive airway pressure, CPAP, bilevel positive airway pressure, BiPAP, or automatic positive airway pressure, APAP) and a suitable subject interface (e.g. a nasal pillow, an oronasal/full-face mask).

Sleep staging is helpful in the diagnosis or during the treatment of SDB. When performing sleep staging, it is determined how much time a subject sleeps in a certain sleep stage. Most known classifications for sleep stages have at least a Wake-class, at least one light sleep class (N1, N2), at least one deep sleep class (N3), and at least one REM sleep class, where REM stands for Rapid Eye Movement. Sleep staging provides clinicians with valuable information about sleep problems and about the efficacy of SDB therapy.

The gold standard measurements for sleep staging are based on measuring neurological activity, which reflect activity of the central nervous system. These measurements require electrodes to be placed on the subject to perform electroencephalography, EEG, electrooculography, EOG, or chin electromyography, EMG. The electrodes, and the wires attached to the electrodes, make such measurements cumbersome for the subject. Also, a clinician needs to apply the electrodes to the correct positions on the subject. Therefore, these measurements are not very well suited to be used over an extended period of time or for use at home.

Instead of the central nervous system, some sleep stage classifiers are based on the autonomic nervous system. The autonomic nervous system plays an important role in regulating involuntary physiological functions, including heart rate, blood pressure, and respiratory rate. During sleep, the autonomic nervous system undergoes significant changes that reflect the different sleep stages. From wake to sleep, the autonomic regulation gradually shifts towards increased parasympathetic tone and sympathetic inhibition. When non-REM sleep deepens from N1 to N2 and further to N3 sleep, parasympathetic dominance continues to grow. During REM sleep, the autonomic balance destabilizes, and parasympathetic activation alternates with burst of sympathetic activity (Stein & Pu, 2012; Tobaldini, et al., 2013). Autonomic nervous system activity can be determined by measuring heart rate, heart rate variability, or respiratory patterns.

However, the mapping between the central nervous system activity and autonomic nervous system activity is an important determinant of the strength of the expression of autonomic nervous system patterns during different sleep stages. This mapping, the so-called autonomic modulation, can differ between subjects and populations. Age, stress and the use of drugs and medicines can have an impact on the autonomic nervous system expression of sleep. Importantly, the presence of sleep disorders can also affect the autonomic modulation (de Zambotti, Trinder, Silvani, Colrain, & Baker, 2018), in particular in obstructive sleep apnea (OSA).

### SUMMARY OF THE INVENTION

Sleep stage classifiers that are based on the autonomic nervous system are trained to a certain population. For example, when a sleep stage classifier is mainly trained on healthy, young individuals, such as in most consumer-oriented sleep tracking devices, the sleep stage classifier may be less accurate in sleep staging for older and sleep-disordered subjects because the model is not tuned to their autonomic modulation. Vice versa, if a sleep stage classifier is trained on older, sleep-disordered populations, the sleep stage classifier may be less accurate in sleep staging for younger, healthy individuals. In case, the sleep stage classifier is trained on both younger and older people, the sleep stage classifier may be less accurate in sleep staging for individuals, either young or old, presenting some degree of autonomic dysfunction or for which the expression of sleep stages in changes of autonomic activity is dampened or somehow altered. The autonomic dysfunction is, for example, caused by pathologies, or medication.

It is an objective of the invention to improve sleep staging based on measurements of physiological properties relating to the autonomic nervous system.

According to a first specific aspect, there is provided a computer-implemented method for sleep staging of a subject, comprising:
receiving a first measurement data representative of a first physiological property relating to the autonomic nervous system of the subject;
providing a first sleep stage classification by using a first sleep stage classifier to classify sleep stages of the subject based on the first measurement data;
providing a second sleep stage classification by using a second sleep stage classifier, wherein the second sleep stage classifier is different than the first sleep stage classifier;
determining a difference between a first performance of the first sleep stage classifier and a second performance of the second sleep stage classifier;
determining, based on the difference, an autonomic modulation value representative of an autonomic modulation of the subject,
wherein the autonomic modulation represents a relationship between the autonomic nervous system and a central nervous system of the subject.

The first sleep stage classifier provides the first sleep stage classification based on measurements of the autonomic nervous system activity. The second sleep stage classifier is different than the first sleep stage classifier and provides the second sleep stage classification. Because the first sleep stage classifier and the second sleep stage are different, the first performance of the first sleep stage classifier may be different than the second performance of the second stage classifier. This difference is a measure for the autonomic modulation, which is the relationship between the autonomic nervous system and a central nervous system of the subject. In case the difference is small, the autonomic modulation of the subject matches with the autonomic modulation on which the first sleep stage classifier is based. For example, the autonomic modulation of the subject matches with the average autonomic modulation of the population on which the first sleep stage classifier is trained. Because the autonomic modulation of the subject matches with the autonomic modulation on which the first sleep stage classifier, the first sleep stage classifier provides reliable sleep stage classification. In case the difference is large, the autonomic modulation of the subject does not match with the autonomic modulation on which the first sleep stage classifier is based. For example, the autonomic modulation of the subject is very different than the average autonomic modulation of the population on which the first sleep stage classifier is trained. Because the autonomic modulation of the subject does not match with the autonomic modulation on which the first sleep stage classifier, the first sleep stage classifier provides unreliable sleep stage classification. Based on the autonomic modulation value, it becomes clear whether the first sleep stage classifier is reliable or not. As a result, improved sleep staging is achieved based on measurements of physiological properties relating the autonomic nervous system.

When performing sleep staging, a sleep session of the subject is divided into epochs. An epoch is, for example, a time interval of 30 seconds. Each epoch is classified to one of the sleep stage classes. The classified epochs are, for example, used to create a hypnogram. A hypnogram represents the sleep stages as a function of time.

For example, the sleep stages are classified into two sleep stage classes, such as a wake sleep stage class and a non-wake sleep stage class, or such as two non-wake sleep stage classes. The two non-wake sleep stage classes are, for example, deep sleep and REM sleep. During the wake sleep stage, the subject is awake. During the non-wake sleep stage, the subject is asleep. For example, the sleep stages are classified into three classes. The three classes are a wake sleep stage class, a REM sleep stage class, and a non-REM sleep stage class. During the REM sleep stage class, the subject is asleep and has Rapid Eye Movements, whereas during the non-REM sleep stage class, the subject is asleep without Rapid Eye Movements. For example, the sleep stages are classified into four classes. The four classes are a wake sleep stage class, a REM sleep stage class, a light sleep stage class, and a deep sleep stage class. For example, the sleep stages are classified into the classes N1, N2, N3, REM and Wake.

The computer-implemented method comprises receiving the first measurement data.

The first measurement data is representative of the first physiological property relating to the autonomic nervous system. For example, the first physiological property relates to a change in the autonomic nervous system activity is. The change in the autonomic nervous system activity is, for example, detected based on cardiac signals obtained with an appropriate sensor such as a reflective photoplethysmography (PPG) sensor, a transmissive PPG sensor or a remote PPG sensor, a ballistocardiographic sensor, or a seismocardiographic sensor. The reflective PPG sensor is, for example, arranged on the wrist or the face of the subject. The transmissive PPG sensor is, for example, arranged on the finger of the subject. The remote PPG sensor comprises, for example, an infrared camera. The ballistocardiographic sensor comprises, for example, an accelerometer or gyroscope attached to the body of the subject, or for example a pressure sensor mounted in the mattress or bed of the subject. The seismocardiographic sensor comprises, for example, an accelerometer mounted on the chest of the subject. The seismocardiographic sensor is, for example, at a distance from the subject and comprises, for example, a radio frequency based sensor, or a laser based sensor.

In addition or alternatively, the first physiological property is, for example, based on respiratory activity. Respiratory activity of the subject is indicative of changes in autonomic nervous system activity associated with different sleep stages. Respiratory activity is, for example, measured with a sensor adapted to measure airflow or adapted to measure chest movements. For example, a sensor adapted to measure airflow comprises an oral cannula, a nasal cannula and/or a thermistor. For example, a sensor adapted to measure chest movements comprises a respiratory inductance plethysmography belt to be worn around the thorax of the abdomen. For example, the sensor adapted to measure respiratory activity comprises a pressure sensor mounted on the bed or mattress. For example, the sensor adapted to measure respiratory activity comprises a Doppler radar positioned near the subject. For example, the sensor for measuring respiratory activity comprises an accelerometer or a gyroscope mounted on the thorax, the abdomen and/or sternum of the subject. For example, the sensor to measure respiratory activity comprises a flow sensor and/or a pressure sensor arranged in a respiratory support device.

The first sleep stage classifier is used to provide the first sleep stage classification. For example, the first sleep stage classifier classifies each epoch to one of the sleep stage classes. For example, the first sleep stage classifier provides multiple probabilities for each epoch, one probability for each sleep stage class. The epoch is classified to the sleep stage class that corresponds to the highest probability. For example, the first sleep stage classifier is trained on a population of subjects. For example, the first sleep stage classifier comprises a machine learning algorithm. For example, the first sleep stage classifier comprises a neural network.

The second sleep stage classifier is different than the first sleep stage classifier. For example, the second sleep stage classifier provides the second sleep stage classification based on the same measurement data as the first sleep stage classifier, i.e., the first measurement data. For example, the second sleep stage classifier provides the second sleep stage classification based on other measurement data than the first sleep stage classifier. The other measurement data is, for example, based on activity of the autonomic nervous system. The other measurement data is, for example, based on activity of the central nervous system. For example, the second sleep stage classifier classifies epochs using the same sleep stage classes as the first sleep stage classifier. For example, the second sleep stage classifier provides multiple probabilities for each epoch, one probability for each sleep stage class. The epoch is classified as the sleep stage class that corresponds to the highest probability. For example, the second sleep stage classifier is trained on the same population of subjects as the first sleep stage classifier, or on a different population than the first sleep stage classifier. For example, the second sleep stage classifier is trained on a population having SDB. For example, the second sleep stage classifier comprises a machine learning algorithm. For example, the second sleep stage classifier comprises a neural network.

The first performance and the second performance are, for example, based on the overall sleep stage performance, for example the sleep stage performance of an entire sleep session. The first performance and the second performance are, for example, based on the sleep stage specific performance, i.e., the performance for the classification of each epoch individually. For example, the first performance and the second performance are based on overall sleep staging performance over a whole night. For example, the first performance and the second performance are based on sleep stage specific performance so over specific parts of the night. For example, the first performance and the second performance are based on specific, individual epochs such as specific transition epochs, e.g., when the subject falls asleep for the first time.

The autonomic modulation value represents the autonomic modulation of the subject. For example, in case the autonomic modulation value does not exceed a threshold, the autonomic modulation of the subject sufficiently matches with the autonomic modulation on which the first sleep stage classifier is based. For example, in case the autonomic modulation value exceeds the threshold, the autonomic modulation of the subject substantially deviates from the autonomic modulation on which the first sleep stage classifier is based. For example, autonomic modulation value exceeds the threshold in case the autonomic modulation value is a positive value. The autonomic modulation value is determined by subtracting a value representing the second performance from a value representing the first performance. Thus, a positive number means an increase in performance of the second sleep stage classifier compared to the first sleep stage classifier. Thus, the autonomic modulation of the subject does not match well the first sleep stage classifier. For example, there is a distribution of the first performance, and/or a distribution of the second performance and/or a difference in a distribution of the difference between the first performance and the second performance obtained during training on a population of the first sleep stage classifier or the second sleep stage classifier. In case the first performance and/or the second performance for the subject falls outside a range of the distribution, e.g., percentile 20%, or percentile 90%, the automatic modulation value exceeds the threshold. The performance of the examples above is, for example based on the Kappa score, F1-score, sensitivity, specificity, accuracy, of overall performance or of sleep stage specific performance. The performance is, for example, based on precision (also referred to as positive predictive value), recall (also referred to as sensitivity), area under the curve, negative predictive value, false positive rate, false negative rate, or any combination thereof. For example, the performance is based on whether an individual epoch is classified correctly or incorrectly. Either the raw number from the examples can be used or a value normalized between 0 and 1.

In an embodiment, the computer-implemented method comprises providing the second sleep stage classifier by personalizing the first sleep stage classifier to the subject.

According to the embodiment, the first sleep stage is personalized to the user to create the second sleep stage classifier. The personalization is done by optimizing the first sleep stage classifier to achieve an improved performance. The personalization that achieves the optimum performance is used to create the second sleep stage classifier. As the second sleep stage classifier is personalized to the subject, the second performance is likely higher than the first performance. If the difference between the first performance and the second performance is low, personalizing of the first sleep stage classifier did not provide a better result. This shows that the first sleep stage classifier is based on an autonomic modulation matching with the subject. If the difference between the first performance and the second performance is high, personalizing of the first sleep stage classifier brings a greatly improved result. This shows that the first sleep stage classifier is based on an autonomic modulation that does not match with the subject. In this case, the first sleep stage classification is unreliable. In another situation, the difference is low, whereas it is expected that the personalization would improve the second performance. This may indicate that the subject suffers from an autonomic impairment or autonomic dysfunction.

In an embodiment, the computer-implemented method comprises receiving subject-specific data about the subject. The computer-implemented method comprises providing the second sleep stage classifier by personalizing the first sleep stage classifier to the subject based on the subject-specific data.

According to the embodiment, the subject-specific data is used to personalize the first sleep stage classifier to create the second sleep stage classifier. The subject-specific data comprises information about the subject, such as age, medication use, apnea hypopnea index AHI, body mass index BMI, or SDB information. For example, the second sleep stage classifier has a neural network with an additional layer compared to the first sleep stage classifier. The additional layer comprises the subject-specific information. For example, the second sleep stage classifier is created by adjusting the softmax function of the first sleep stage classifier based on the subject-specific data.

In an embodiment, the computer-implemented method comprises receiving second measurement data representative of a second physiological property relating to the central nervous system of the subject. The computer-implemented method comprises providing the second sleep stage classification by using the second sleep stage classifier based on the second measurement data.

According to the embodiment, the second sleep stage classifier uses measurement data based on the central nervous system. As a result, the difference between the first performance and the second performance is based on measurement data from the autonomic nervous system as well as measurement data from the central nervous system. This allows the autonomic modulation to be determined with improved accuracy. For example, the first measurement data is obtained every night, whereas the second measurement data is obtained only once a month or once every half year or once every year. This way, any changes to the subject that affects the autonomic modulation, such as a progression or a change in a medical condition of the subject, can be detected based on the second measurement data.

In an embodiment, the second measurement data comprises one or more of electroencephalography (EEG) data, electrooculography (EOG) data, and electromyography (EMG) data.

In an embodiment, the computer-implemented method comprises providing the second sleep stage classifier by adjusting the first sleep stage classifier based on the first measurement data and the second measurement data.

According to the embodiment, the second sleep stage classifier is created by adjusting the first sleep stage classifier. For example, the second sleep stage classifier is created by fine-tuning the first sleep stage classifier based on the first measurement data and the second measurement data. The first measurement data and the second measurement data are concurrent data. For example, the second sleep stage classifier is created by model adaptation of the first sleep stage classifier. For example, the model adaptation is done without any model retraining. For example, the model adaptation makes use of Kullbach-Leibler divergence. The Kullbach-Leibler divergence is, for example, performed on the sleep stage classification each epoch, or on the probabilities for each sleep stage class for each epoch.

In an embodiment, the physiological property comprises a cardiac property or a respiratory property of the subject.

In an embodiment, determining the difference comprises performing a comparison between sleep stages according to the first sleep stage classification and sleep stages according to the second sleep stage classification, and determining the difference based on the comparison.

According to the embodiment, the difference is determined based on how the epochs are classified by the first sleep stage classifier and how the epochs are classified by the second sleep stage classifier. In case a large portion of the epochs are classified the same by both the first sleep stage classifier and the second sleep stage classifier, the autonomic modulation of the subject matches with the autonomic modulation on which the first sleep stage classifier is based. In case only a small portion of the epochs are classified the same by both the first sleep stage classifier and the second sleep stage classifier, the autonomic modulation of the subject does not match well with the autonomic modulation on which the first sleep stage classifier is based.

In an embodiment, the comparison is based on a probability of sleep stages according to the first sleep stage classification and based on a probability of sleep stages according to the second sleep stage classification.

According to the embodiment, the first sleep classifier provides the first sleep stage classification with for each epoch probabilities for all sleep stage classes. The second sleep classifier provides the second sleep stage classification with for each epoch probabilities for all sleep stage classes. The comparison is based on the multiple probabilities for each epoch. In case a large portion of the probabilities are the same as classified by the first sleep stage classifier and the second sleep stage classifier, the autonomic modulation of the subject matches with the autonomic modulation on which the first sleep stage classifier is based. In case only a small portion of the probabilities are the same as classified by the first sleep stage classifier and the second sleep stage classifier, the autonomic modulation of the subject does not match well with the autonomic modulation on which the first sleep stage classifier is based.

In an embodiment, the computer-implemented method comprises determining the first performance and the second performance based on one or more of a Cohen's kapa coefficient, an F1-score, and an accuracy.

In an embodiment, the computer-implemented method comprises providing, based on the autonomic modulation value, a reliability value representative of a reliability of the first sleep stage classifier.

The autonomic modulation value based on a small difference represents that the autonomic modulation of the first sleep stage classifier matches with the autonomic modulation of the subject. As a result, the sleep stage classification by the first sleep stage classifier provides reliable results. The autonomic modulation value based on a large difference represents that the autonomic modulation of the first sleep stage classifier does not match with the autonomic modulation of the subject. As a result, the sleep stage classification by the first sleep stage classifier provides unreliable results.

In an embodiment, the computer-implemented method comprises determining, based on the autonomic modulation value, a change in the relationship between the autonomic nervous system and the central nervous system of the subject.

According to this embodiment, the autonomic modulation value is determined multiple times over a time period, while using the first sleep stage classifier and the second sleep stage classifier. Without any changes to the first sleep stage classifier and the second sleep stage classifier, a change in the autonomic modulation value is representative of a change in the relationship between the autonomic nervous system and the central nervous system of the subject. Such a change provides valuable clinical information about the subject. For example, a medical condition, such as sleep related condition as SDB or insomnia, has improved or worsened. For example, the change in the relationship shows that an applied SDB therapy or an applied insomnia therapy is effective. For example, the SDB therapy comprises positive airway pressure, PAP, therapy. For example, the insomnia therapy comprises cognitive behavioral therapy for insomnia, CBT-i. For example, the change in the relationship shows a medication used by the subject has an improved effect or a reduced effect on the subject.

According to a second aspect of the invention, there is provided a computer program product, comprising instructions which, when executed by a processor, cause the computer-implemented method according to the first aspect to be carried out.

According to a third aspect of the invention, there is provided a computer-readable storage medium comprising instructions which, when executed by a processor, cause the computer-implemented method according to the first aspect to be carried out.

According to a fourth aspect of the invention, there is provided a sleep staging device adapted to perform sleep staging for a subject. The sleep staging device comprises a processor configured to cause the computer-implemented method according to the first aspect to be carried out. The sleep staging device comprises a sensor input adapted to receive from a sensor the first measurement data. The sleep staging device comprises an output unit adapted to generate an output signal representative of the autonomic modulation value.

In an embodiment, the sleep staging device comprises a photoplethysmography, PPG, sensor, adapted to generate PPG data representative of blood volume changes of the subject. The sleep staging device comprises an actigraphy sensor adapted to generate activity data representative of gross motor activity of the subject. The first measurement data comprises the PPG data and the activity data.

For example, the actigraphy sensor comprises an accelerometer to detect accelerations of the sleep staging device. For example, the sleep staging device is a wearable device, for example, to be attached to the subject at the wrist or at the chest or at the head or at the abdomen.

### BRIEF DESCRIPTION OF THE DRAWINGS

Exemplary embodiments will now be described, by way of example only, with reference to the following figures, in which:
FIG. 1 depicts a first embodiment according to the invention;
FIG. 2 depicts a second embodiment according to the invention;
FIG. 3 depicts a third embodiment according to the invention;
FIG. 4 depicts a fourth embodiment according to the invention;
FIG. 5 depicts a fifth embodiment according to the invention;
FIG. 6 depicts a sixth embodiment according to the invention;
FIGs. 7 and 8 depict the results of a study making use of the computer-implemented method.

### DETAILED DESCRIPTION OF THE EMBODIMENTS

It should be understood that the detailed description and specific examples, while indicating exemplary embodiments of the apparatus, systems and methods, are intended for purposes of illustration only and are not intended to limit the scope of the invention. These and other features, aspects, and advantages of the computer-implemented methods and devices of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

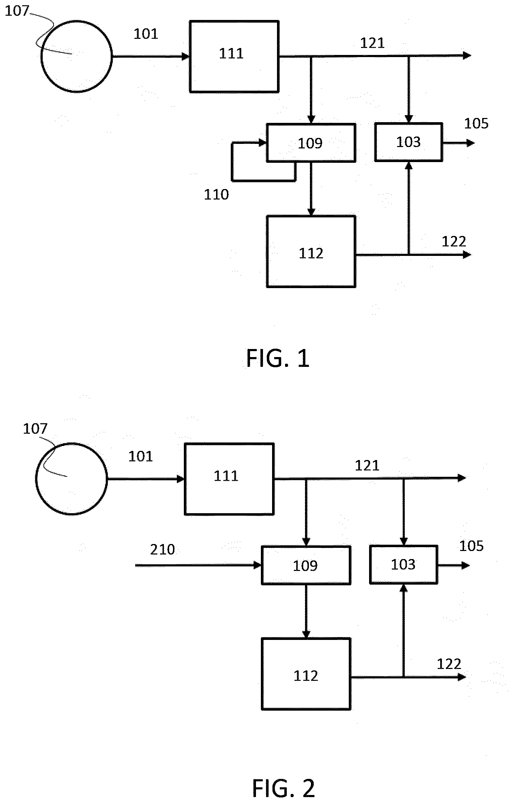

FIG. 1 depicts a first embodiment of the computer-implemented method according to the invention. In the first embodiment, the computer-implemented method is for sleep staging of a subject. The computer-implemented method comprises receiving a first measurement data 101 representative of a first physiological property relating to the autonomic nervous system of the subject. The computer-implemented method comprises providing a first sleep stage classification by using a first sleep stage classifier 111 to classify sleep stages of the subject based on the first measurement data 101. The computer-implemented method comprises providing a second sleep stage classification by using a second sleep stage classifier 112. The second sleep stage classifier 112 is different than the first sleep stage classifier 111. The computer-implemented method comprises determining a difference 103 between a first performance of the first sleep stage classifier 111 and a second performance of the second sleep stage classifier 112. The computer-implemented method comprises determining, based on the difference 103, an autonomic modulation value 105 representative of an autonomic modulation of the subject. The autonomic modulation represents a relationship between the autonomic nervous system and a central nervous system of the subject.

The first sleep stage classifier 111 provides the first sleep stage classification via a first output 121. The second sleep stage classifier 112 provides the second sleep stage classification via a second output 122. The computer-implemented method uses the first output 121 and the second output 122 to determine the difference 103.

The computer-implemented method comprises providing the second sleep stage classifier 112 by personalizing the first sleep stage classifier 111 to the subject. The personalizing is done at 109, at which parameters of the first sleep stage classifier 111 are adjusted in an attempt to provide sleep stage classification with an improved performance. For example, the parameters comprise weighing factors, or features, or attributes of the first sleep stage classifier 111. For example, the personalizing at 109 includes multiple iterations 110 of adjusting parameters of the first sleep stage classifier 111. The iterations 110 stop in case a maximum number of iterations 110 is reached, or in case a sufficient amount of improvement is achieved. The second classifier is created based on the adjusted parameters of the first sleep stage classifier 111. For example, the personalizing the first sleep stage classifier 111 to the subject is based on fine-tuning or model adaption of the first sleep stage classifier 111.

The physiological property comprises, for example, a cardiac property or a respiratory property of the subject. The first measurement data 101 comprises information about the cardiac property or the respiratory property.

The computer-implemented method comprises, for example, determining the first performance and the second performance based on one or more of a Cohen's kapa coefficient, an F1-score, and an accuracy.

For example, the computer-implemented method comprises providing, based on the autonomic modulation value 105, a reliability value representative of a reliability of the first sleep stage classifier 111.

For example, the computer-implemented method comprises determining, based on the autonomic modulation value 105, a change in the relationship between the autonomic nervous system and the central nervous system of the subject.

For example, determining the difference 103 comprises performing a comparison between sleep stages according to the first sleep stage classification and sleep stages according to the second sleep stage classification. The difference 103 is determined based on the comparison.

For example, the comparison is based on a probability of sleep stages according to the first sleep stage classification and based on a probability of sleep stages according to the second sleep stage classification.

FIG. 2 depicts a second embodiment according to the invention. The second embodiment has, for example, the same features as the first embodiment, except for the following.

In the second embodiment, the computer-implemented method comprises receiving subject-specific data 210 about the subject. The computer-implemented method comprises providing the second sleep stage classifier 112 by personalizing the first sleep stage classifier 111 to the subject based on the subject-specific data 210. The personalizing at step 109 is done by using subject-specific data 210, such as age, or medical information of the subject, or gender, or medication use of the subject.

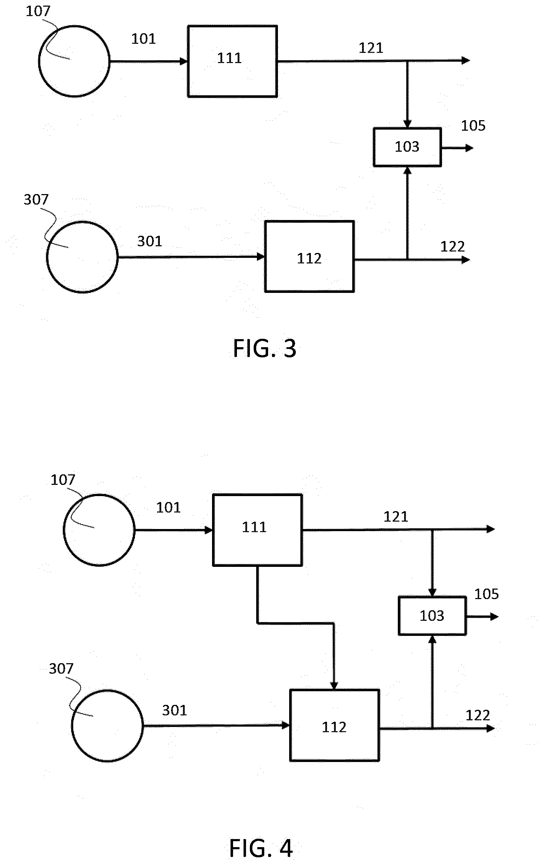

FIG. 3 depicts a third embodiment according to the invention. The third embodiment has, for example, the same features as the first embodiment or the second embodiment, except for the following.

In the third embodiment, the computer-implemented method comprises receiving second measurement data 301 representative of a second physiological property relating to the central nervous system of the subject. The computer-implemented method comprises providing the second sleep stage classification by using the second sleep stage classifier 112 based on the second measurement data 301.

For example, the second measurement data 301 comprises one or more of electroencephalography (EEG) data, electrooculography (EOG) data, and electromyography (EMG) data.

In the third embodiment, the second sleep stage classifier 112 is adapted to perform the second sleep stage classification based on information of the central nervous system of the subject.

FIG. 4 depicts a fourth embodiment according to the invention. The fourth embodiment has, for example, the same features as the first embodiment, the second embodiment, or the third embodiment, except for the following.

In the fourth embodiment, the computer-implemented method comprises providing the second sleep stage classifier 112 by adjusting the first sleep stage classifier 111 based on the first measurement data 101 and the second measurement data 301.

For example, parameters of the first sleep stage classifier 111 are adjusted based on the second measurement data 301 to create the second sleep stage classifier 112. For example, the second sleep stage classifier 112 comprises part of the first sleep stage classifier 111.

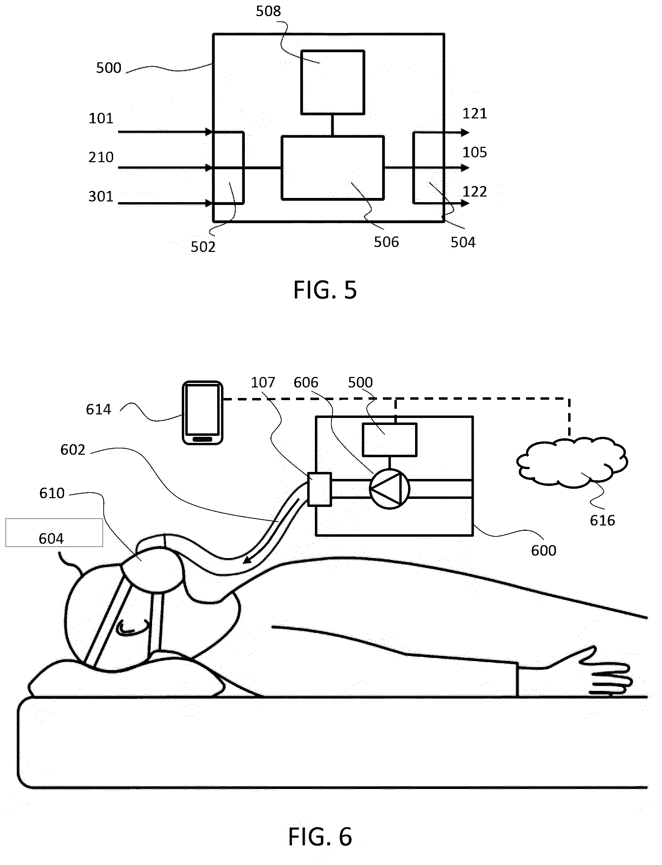

FIG. 5 depicts a fifth embodiment according to the invention. The fifth embodiment has, for example, the same features as the first embodiment, the second embodiment, the third embodiment, or the fourth embodiment except for the following.

In the fifth embodiment, a processor 506 is provided. The processor 506 is configured to cooperate with a sensor input 502, an output unit 504, and a memory 508. The memory 508 is a computer-readable storage medium. The memory 508 stores a computer program product. The computer program product comprises instructions which, when executed by the processor 506, causes the computer-implemented method according to any one the embodiments to be carried out.

The sensor input 502 is adapted to receive the first measurement data 101. Depending on the embodiment, the sensor input 502 is adapted to receive the subject-specific data 210, and/or the second measurement data 301.

Optionally, the processor 506 is part of a sleep staging device 500. The sleep staging device 500 is adapted to perform sleep staging for a subject. The sleep staging device 500 comprises the processor 506 configured to cause the computer-implemented method according to any one of the embodiments to be carried out. The sleep staging device 500 comprises the sensor input 502 adapted to receive from the sensor 107 the first measurement data 101. The sleep staging device 500 comprises the output unit 504 adapted to generate an output signal representative of the autonomic modulation value 105. Optionally, the output unit 504 outputs the first output 121 and/or the second output 122.

Optionally, the sleep staging device 500 comprises a photoplethysmography, PPG, sensor, adapted to generate PPG data representative of blood volume changes of the subject, and an actigraphy sensor adapted to generate activity data representative of gross motor activity of the subject. The first measurement data 101 comprises the PPG data and the activity data.

FIG. 6 depicts a sixth embodiment according to the invention. The sixth embodiment has, for example, the same features as the first embodiment, the second embodiment, the third embodiment, fourth embodiment, or the fifth embodiment, except for the following.

In the sixth embodiment, there is provided a respiratory support device 600 adapted to provide a pressurized airflow to a subject 604. The respiratory support device 600 comprises a pressure source 606 adapted to generate the pressurized airflow 602. The respiratory support device 600 comprises the sleep staging device 500 according to fifth embodiment. The sensor 107 is adapted to generate the first measurement data 101 representative of a property of the pressurized airflow. The respiratory support device 600 comprises the sensor 107.

The subject uses a patient interface 610, such as a mask, to receive the pressurized airflow 602. The sensor 107 is arranged along the path of the pressurized airflow 602. The sensor 107 is adapted to generate the first measurement data 101. The first measurement data 101 is representative of a pressure and/or a flow of the pressurized airflow 602.

Optionally, the sleep staging device 500 is adapted to communicate with a mobile device 614, such as a mobile phone. The sleep staging device 500 is adapted, for example, to provide the autonomic modulation value 105 to the mobile device 614, causing the mobile device 614 to display the autonomic modulation value 105.

Optionally, the sleep staging device 500 is adapted to communicate with distant processor, for example a distant processor arranged in the cloud 616. The sleep staging device 500 is adapted to cause the distant processor to perform part of the computer-implemented method.

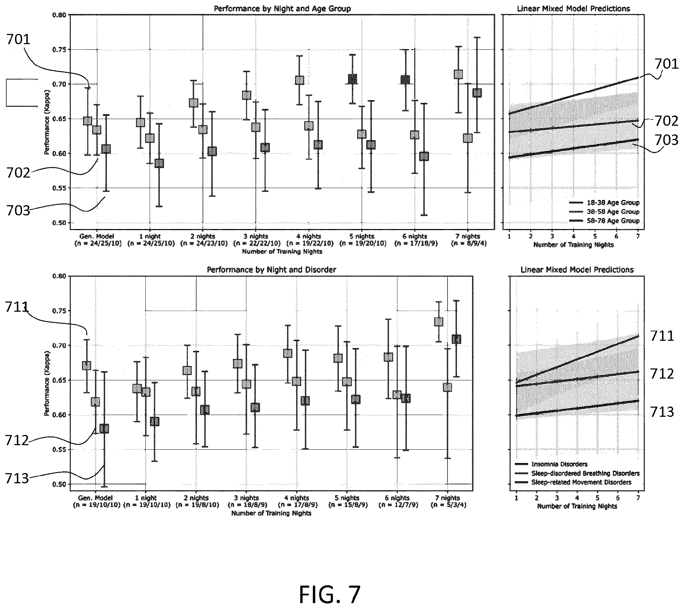

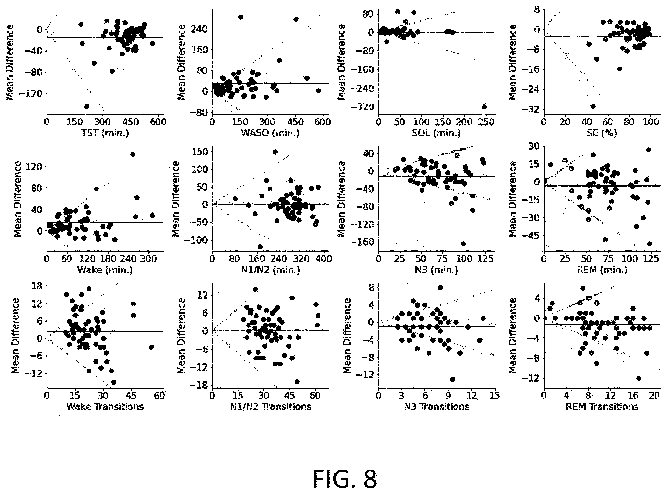

FIGs. 7 and 8 depict the results of a study making use of the computer-implemented method.

FIG. 7 depicts in the upper graph, the performance of the computer-implemented method. At the left of the figure, the performance is shown for the generic model that is used without any personalization to the subject. The generic model comprises the first sleep stage classifier 111. The other performances are shown for 1-7 nights of personalization. The other performances are based on the second performance of the second sleep stage classifier 112 after 1-7 nights of personalization. Each performance is shown for three different age groups, group 701 shows the age 18-38, group 702 shows the age 38-58, and group 703 shows the age 58-78.

FIG. 7 depicts in the lower graph, the performance of the computer-implemented method per night and per sleep related disorder. Each performance is shown for three different groups of sleep related disorders. Group 711 shows the performance for subjects with insomnia disorders. Group 712 shows the performance for subjects with SDB disorders. Group 713 shows the performance for subjects with sleep-related movement disorders.

FIG. 7 shows the effect of number of training nights on the personalized performance (Kappa) for the different age groups (upper graph) and the different sleep disorders (lower graph). In the left plot, the error bars represent group means ± standard deviations. In the right plot, the solid lines represent the LMM predicted performance, including the 95% confidence interval of the prediction. The data distributions (left) and predictions (right) do not necessarily align since the prediction considers repeated measures. Note that personalization on more nights holds less predictive power due to decreasing sample sizes. Sample sizes are reported in x-axis labels for the 18-38, 38-58, and 58-78 age groups, and for insomnia disorders, sleep-disordered breathing, and sleep-related movement disorders, respectively.

In the study, there is a significant interaction between the number of training nights and the 38-58 age group, β = -.007, 95% CI = [-.013, -.000], SE = .003, z = -2.09, p = .037, as well as for the 58-78 age group, β = -.007, 95% CI = [-.013, -.000], SE = .003, z = -2.09, p = .037, indicating that the increase in performance with additional training was lower when compared to the younger reference 18-38 years group. Hence, for younger subjects, there were subject-specific autonomic representations that were learned through personalization, suggesting the autonomic modulation in these younger individuals was different than in the representation in the first sleep stage classifier 111.

Moreover, there was a significant interaction between the number of training nights and the sleep-disordered breathing population, β = -.004, 95% CI = [-.008, -.000], SE = .002, z = -2.23, p = .03, indicating that the increase in performance with additional training nights is lower when compared in the insomnia population. Hence for patients with insomnias, there were subject-specific autonomic representations that were learned through personalization, suggesting the autonomic modulation in these patients with insomnia was different than in the representation in the first sleep stage classifier 111.

FIG. 8 depicts Bland-Altman plots for twelve sleep macrostructure parameters, computed by subtracting the mean of the second sleep stage classifier 112 from the generalized model, i.e., the first sleep stage classifier 111. For each subject, the second sleep stage classifier 112 which included all available home recordings was selected. Four sleep statistics (in minutes), time spent in each sleep stage (in minutes), and the number of transitions to each sleep stage are shown. Solid lines indicate mean differences, and the gray areas indicate the 95% limits of agreement. Moreover, areas above and under dotted lines indicate where the relative difference between the first sleep stage classifier 111 and the second sleep stage classifier 112 is larger than 50%. The second output 122 from the second sleep stage classifier 112 was subtracted from the first output 121 of the first sleep stage classifier 111, hence negative mean differences indicate higher sleep statistic values in the second sleep stage classifier 112. The abbreviations in FIG. 8 are as follows: TST: total sleeping time; WASO: wake after sleep onset; SE: sleep efficiency; and SOL: sleep onset latency.

Also, there can be large differences in the detection of sleep stages (subsequently derived sleep statistics) between the first sleep stage classifier 111 and second sleep stage classifier 112, where differences on specific metrics can 50% or more. When the second sleep stage classifier 112 has been validated, the absolute and relative differences in these metrics can be used as marker to define whether there is presence of altered autonomic modulation without the need of a gold-standard evaluation with manual scoring.

It will be understood that the disclosed methods are computer-implemented methods. As such, there is also proposed a concept of a computer program comprising code means for implementing any described method when said program is run on a processing system.

The skilled person would be readily capable of developing a processor 506 for carrying out any herein described method. Thus, each step of a flow chart may represent a different action performed by a processor 506, and may be performed by a respective module of the processor 506.

As discussed above, the system makes use of a processor 506 to perform the data processing. The processor 506 can be implemented in numerous ways, with software and/or hardware, to perform the various functions required. The processor 506 typically employs one or more microprocessors that may be programmed using software (e.g. microcode) to perform the required functions. The processor 506 may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs). Thus, the processor 506 may be embodied as a digital and/or analog processor 506.

In various implementations, the processing system may be associated with one or more storage media such as volatile and non-volatile computer memory 508 such as RAM, PROM, EPROM, and EEPROM. The storage media may be encoded with one or more programs that, when executed on one or more processors and/or controllers, perform the required functions. Various storage media may be fixed within a processing system or controller may be transportable, such that the one or more programs stored thereon can be loaded into a processor 506.

Functions implemented by a processor 506 may be implemented by a single processor 506 or by multiple separate processing units which may together be considered to constitute a "processor 506". Such processing units may in some cases be remote from each other and communicate with each other in a wired or wireless manner.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A computer-implemented method for sleep staging of a subject, comprising:
receiving a first measurement data (101) representative of a first physiological property relating to the autonomic nervous system of the subject;
providing a first sleep stage classification by using a first sleep stage classifier (111) to classify sleep stages of the subject based on the first measurement data (101);
providing a second sleep stage classification by using a second sleep stage classifier (112), wherein the second sleep stage classifier (112) is different than the first sleep stage classifier (111);
determining a difference (103) between a first performance of the first sleep stage classifier (111) and a second performance of the second sleep stage classifier (112);
determining, based on the difference (103), an autonomic modulation value (105) representative of an autonomic modulation of the subject,
wherein the autonomic modulation represents a relationship between the autonomic nervous system and a central nervous system of the subject.

2. The computer-implemented method according to claim 1, comprising
providing the second sleep stage classifier (112) by personalizing the first sleep stage classifier (111) to the subject.

3. The computer-implemented method according to claim 2, comprising
receiving subject-specific data (210) about the subject; and
providing the second sleep stage classifier (112) by personalizing the first sleep stage classifier (111) to the subject based on the subject-specific data (210).

4. The computer-implemented method according to claim 2 or 3, comprising:
receiving second measurement data (301) representative of a second physiological property relating to the central nervous system of the subject; and
providing the second sleep stage classification by using the second sleep stage classifier (112) based on the second measurement data (301).

5. The computer-implemented method acceding to claim 4,
wherein the second measurement data (301) comprises one or more of electroencephalography (EEG) data, electrooculography (EOG) data, and electromyography (EMG) data.

6. The computer-implemented method according to claim 4 or 5, comprising
providing the second sleep stage classifier (112) by adjusting the first sleep stage classifier (111) based on the first measurement data (101) and the second measurement data (301).

7. The computer-implemented method according to any one of the preceding claims,
wherein determining the difference (103) comprises:
performing a comparison between sleep stages according to the first sleep stage classification and sleep stages according to the second sleep stage classification; and
determining the difference (103) based on the comparison.

8. The computer-implemented method according to any one of the preceding claims, comprising
determining the first performance and the second performance based on one or more of a Cohen's kapa coefficient, an F1-score, and an accuracy.

9. The computer-implemented method according to any one of the preceding claims, comprising:
providing, based on the autonomic modulation value (105), a reliability value representative of a reliability of the first sleep stage classifier (111).

10. The computer-implemented method according to any one of the preceding claims, comprising:
determining, based on the autonomic modulation value (105), a change in the relationship between the autonomic nervous system and the central nervous system of the subject.

11. A computer program product, comprising instructions which, when executed by a processor (506), cause the computer-implemented method according to any one of the preceding claims to be carried out.

12. A computer-readable storage medium comprising instructions which, when executed by a processor (506), cause the computer-implemented method according to any one of claims 1-10 to be carried out.

13. A sleep staging device (500) adapted to perform sleep staging for a subject, comprising:
a processor (506) configured to cause the computer-implemented method according to any one of claims 1-10 to be carried out;
a sensor (107) input adapted to receive from a sensor (107) the first measurement data (101);
an output unit (504) adapted to generate an output signal representative of the autonomic modulation value (105).

14. A sleep staging device (500) according to claim 13, comprising
a photoplethysmography, PPG, sensor (107), adapted to generate PPG data representative of blood volume changes of the subject;
an actigraphy sensor (107) adapted to generate activity data representative of gross motor activity of the subject;
wherein the first measurement data (101) comprises the PPG data and the activity data.

15. A respiratory support device (600) adapted to provide a pressurized airflow (602) to a subject, comprising:
a pressure source adapted to generate the pressurized airflow (602);
a sleep staging device (500) according to claim 14,
wherein the sensor (107) is adapted to generate the first measurement data (101) representative of a property of the pressurized airflow (602).
